# EUROPEAN PATENT APPLICATION

(11) **EP 1 921 571 A2**
(43) Date of publication of application: **14.05.2008**
(21) Application number: 07008139.3
(22) Date of filing: 20.04.2007
(51) Int. Cl.: G06K 19/077

(54) **Biological implantation RFID tags and insertion jig therefor**

(30) Priority: 07.11.2006 JP 2006302075
(71) Applicant: Hitachi, Ltd., Chiyoda-ku Tokyo 100-8280 (JP)
(72) Inventor: Sakama, Isao, Tokyo 100-8220 (JP); Ashizawa, Minoru, Tokyo 100-8220 (JP); Abe, Hiroshi, Tokyo 100-8220 (JP)
(74) Representative: Strehl Schübel-Hopf & Partner

(57) **Abstract**

The present invention provides a biological implantation RFID tag which can be subcutaneously implanted in a small animal without significantly stressing it and which exhibits improved communication performance, and an insertion jig used to implant the biological implantation RFID tag in the living body of a small animal. A biological implantation RFID tag (1) is implanted in the living body of a small animal to transmit and receive an electromagnetic wave to and from the exterior of the living body to allow an ID assigned to the animal to be read. The biological implantation RFID tag (1) includes an inlet having an IC chip (15) that stores the ID and a main antenna (13), a support (21) around which the inlet is wound to form an inlet portion (10), and an auxiliary antenna (30) connected to the main antenna (13) to extend it. Implanting the biological implantation RFID tag (1) in the living body allows the inlet portion (10) to be implanted in a subcutaneous tissue layer with most of the auxiliary antenna (30) projecting out from the living body.

## Description

### Background of the invention

The present invention relates to biological implantation RFID (Radio Frequency IDentification) tags which can be subcutaneously implanted in small animals without significantly stressing them and which exhibit improved communication performance, and insertion jigs used to implant the biological implantation RFID tags in the living bodies of small animals.

In recent years, RFID tags have been commonly used to label pets, domestic livestock, and experimental animals. The RFID tag includes an IC chip and an antenna and can transmit data such as an ID (IDentification: identifier) recorded in the IC chip, via an RF (Radio Frequency; high frequency) signal through the antenna. Thus, an RFID tag reader enables the ID data recorded in the IC chip to be read in a noncontact and noninvasive manner, allowing the animal labeled by the RFID tag to be identified. Such RFID tags are implanted in the living bodies of animals and thus called biological implantation RFID tags. Biological implantation RFID tags are used to label various animals ranging from big animals such as cow and horses to medium-size animals such as sheep, penguins, dogs, and cats.

For example, a biological element transponder is known which includes a computer microchip in which a unique identifier consisting of 10 to 15 digits is recorded, an antenna coil having a ferrite or iron core around which a copper wire is wound, a tuning capacitor, and a glass capsule which consists of soda glass and which houses the computer microchip, antenna coil, and tuning capacitor (see, for example, Terry Watkins "Is the biochip the Mark of the Beast?" [on line]. Dial the-Truth Ministries, 1999. [retrieved on 2006-10-27]. Retrieved from the Internet: URL: http://www.av1611.org/666/biochip.html). The glass capsule has minimum dimensions including a length of 11 mm and a diameter of 2 mm. For most dogs and cats, the biological element is inserted into the back of the neck between the blade bones.

In general, if foreign matter such as the RFID tag is subcutaneously implanted in an animal, the maximum dimensions of an RFID tag that can be implanted in the animal while avoiding significant stress on the animal to prevent the lifetime of the animal from being affected include a length of at most 10% of the body length of the animal and a diameter of at most 1.5% of the body length. For example, if an RFID tag of length about 12 mm and diameter about 1.6 mm is implanted in the living body of a medium-size animal of body length at least 20 cm (for example, a rat), since the length of the RFID tag is at most 6% of the body length of the medium-size animal, the animal is not significantly stressed. This avoids affecting the lifetime of the animal.

### Brief Summary of the Invention

However, if an RFID tag of length about 12 mm and diameter about 1.6 mm is implanted in the living body of a small animal of body length about 10 cm, the length of the RFID tag is about 12% of the body length of the small animal. The small animal is thus significantly stressed to have its lifetime sharply reduced. A typical example of small animals used for animal experiments is mice. The size of an adult mouse is such that its body length is about 7 to 8 cm (tail length is about 7 cm). The size of the above RFID tag is such that its length is equal to 15 to 17% of the body length of the mouse and such that its diameter is equal to 2.0 to 2.2% of the body length. Consequently, implanting the RFID tag in the mouse significantly stresses the mouse, sharply reducing its lifetime.

Further, the above biological element (see the above prior art document) includes the glass capsule containing the antenna coil or the like having the ferrite or iron core around which the copper wire is wound. Accordingly, it is difficult to reduce the size and weight of the biological element. Furthermore, the cores of the glass capsule and antenna coil are not flexible. This disadvantageously places significant stress on the small animal to sharply reduce its lifetime.

Moreover, the above biological element (see the above prior art document) uses low-frequency radio signals to enable the communication between the implanted biological element and the living body. Thus, the antenna coil needs to be able to transmit and receive low-frequency radio signals, preventing the size and weight of the biological element from being reduced.

Thus, the use of high-frequency radio signals has been proposed. However, in general, an electromagnetic wave of a higher frequency is more significantly attenuated by the living body. Thus, disadvantageously, if an electromagnetic wave of a high frequency, for example, a microwave band, is used for communication, a special reader antenna needs to be used instead of an ordinary tag reader antenna. Further, such an electromagnetic wave provides only a short communication distance and a narrow reading area.

In view of the above problems, an object of the present invention is to provide a biological implantation RFID tag which can be subcutaneously implanted in a small animal without significantly stressing it and which exhibits improved communication performance, and an insertion jig used to implant the biological implantation RFID tag in the living body of a small animal.

To accomplish this object, a biological implantation RFID tag in accordance with the present invention is provided with a unique identifier and implanted in a living body of an animal, and allows the identifier to be read by transmitting an electromagnetic wave between the living body and an exterior. The biological implantation RFID tag includes an inlet having a base material including a dielectric, an IC chip mounted on the base material and storing the identifier, and a main antenna connected to the IC chip, and an auxiliary antenna connected to the main antenna to extend the main antenna.

Further, an insertion jig for a biological implantation RFID tag in accordance with the present invention is used for a biological implantation RFID tag provided with a unique identifier and implanted in a living body of an animal, and allows the identifier to be read by transmitting an electromagnetic wave between the living body and an exterior. The insertion jig includes the biological implantation RFID tag in accordance with the present invention and a suture with a needle integrally combined with the biological implantation RFID tag.

These specific means will be described in detail through embodiments described below.

The present invention can provide a biological implantation RFID (Radio Frequency IDentification) tag which can be subcutaneously implanted in a small animal without significantly stressing it and which exhibits improved communication performance, and an insertion jig used to implant the biological implantation RFID tag in the living body of a small animal.

Other objects, features and advantages of the invention will become apparent from the following description of the embodiments of the invention taken in conjunction with the accompanying drawings.

### Brief Description of the Several Views of the Drawings

Fig. 1 is a diagram showing the structure of a biological implantation RFID tag in accordance with a first embodiment of the present invention;
Fig. 2 is a plan view of an inlet applied to the biological implantation RFID tag in accordance with the first embodiment of the present invention;
Fig. 3 is a perspective view of a support to which the inlet shown in Fig. 2 is stuck;
Fig. 4 is a diagram showing the appearance of the biological implantation RFID tag in accordance with the first embodiment of the present invention before it is covered;
Fig. 5A is a sectional view taken along line VA-VA in Fig. 4 and showing the sectional shape of a first example of the biological implantation RFID tag shown in Fig. 4, and Fig. 5B is a sectional view showing the sectional shape of a second example of the biological implantation RFID tag shown in Fig. 4;
Fig. 6 is a sectional view taken along line VI-VI in Fig. 4 and showing an inlet shown in Fig. 4;
Fig. 7 is a diagram showing the appearance of a support having a recess;
Fig. 8A and B is a diagram showing a biological implantation RFID tag manufactured in accordance with a variation of the first embodiment of the present invention;
Fig. 9A to C is a diagram showing the assembly of a biological implantation RFID tag in accordance with a second embodiment of the present invention;
Fig. 10 is a diagram showing the structure of the biological implantation RFID tag in accordance with the second embodiment of the present invention;
Fig. 11A to F is a diagram showing the assembly and configuration of a biological implantation RFID tag in accordance with a third embodiment of the present invention;
Fig. 12A to E is a diagram showing the assembly and configuration of a biological implantation RFID tag in accordance with a fourth embodiment of the present invention;
Fig. 13A to E is a diagram showing the assembly and configuration of a biological implantation RFID tag in accordance with a fifth embodiment of the present invention;
Fig. 14A and B is a diagram showing the assembly and configuration of a biological implantation RFID tag in accordance with a sixth embodiment of the present invention;
Fig. 15A and B is a perspective view showing a biological implantation RFID tag in accordance with a seventh embodiment of the present invention;
Fig. 16 is a diagram showing the configuration of an insertion jig for a biological implantation RFID tag in a first example of an eighth embodiment of the present invention;
Fig. 17A and B is a diagram showing the configuration of an insertion jig for a biological implantation RFID tag in a second example of the eighth embodiment of the present invention; and
Fig. 18 is a sectional view showing the structure of the skin of a common animal.

### Detailed description of the invention

Embodiments of the present invention will be described below in detail with reference to the accompanying figures. In the description below, substantially the same components are denoted by the same reference numerals, with duplicate descriptions omitted.

### «First Embodiment»

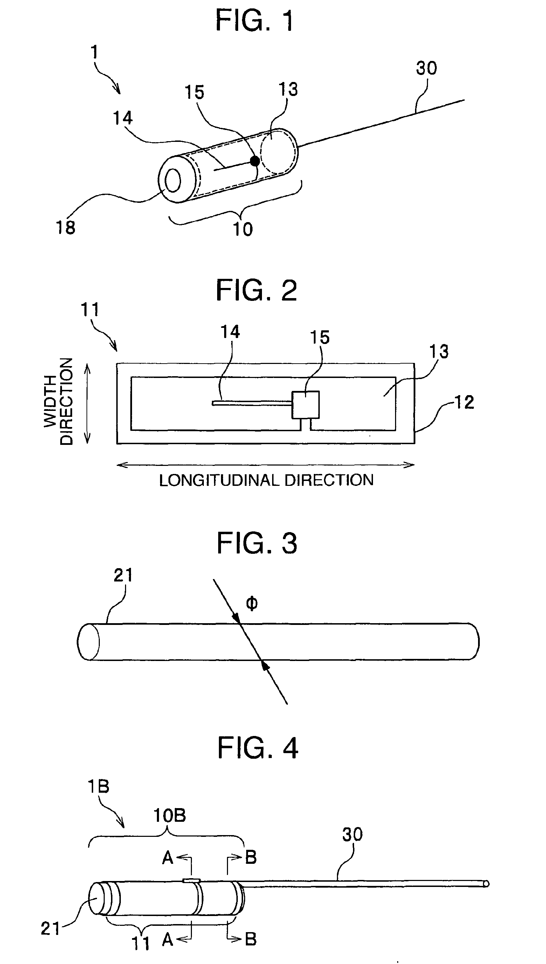

Fig. 1 is a diagram showing the structure of a biological implantation RFID tag 1 in accordance with a first embodiment of the present invention.

The biological implantation RFID tag 1 has an inlet portion 10 functioning as a biological implantation RFID tag and an auxiliary antenna 30 attached to the inlet portion 10. As described below, the entire inlet portion 10 of the biological implantation RFID tag 1 is subcutaneously implanted at a subcutaneous site (described below) of a small animal with all or a part of, preferably, most part of the auxiliary antenna 30 projected toward a free space outside the living body.

The biological implantation RFID tag 1 is subcutaneously implanted in a mouse or the like and is thus very small. However, the biological implantation RFID tag 1 can be implanted in other small or medium-size animals without posing any problem. The size of the biological implantation RFID tag 1 is such that its length is equal to at most 10% of the body length of a mouse and its diameter of at most 1.5% of the body length, so as to prevent the mouse from being significantly stressed when the biological implantation RFID tag is implanted in the mouse. Specifically, since an adult mouse is 7 to 8 cm in body length, the biological implantation RFID tag 1 has a diameter of at most 1 mm and a length of at most 7 mm. To be precise, the size of the biological implantation RFID tag 1 herein means the size of the inlet portion 10. That is, the biological implantation RFID tag 1 is implanted so that most of the auxiliary antenna 30 is located outside the living body.

The inlet portion 10 of diameter 1 mm and length 7 mm is constructed by connecting a main antenna 13 of length 1 mm and width 7 mm to an IC chip 15 (mu-chip (registered trade mark)) to form an inlet 11 (see Fig. 2), wrapping the inlet 11 around a support 21 consisting of a flexible resin so that the resonance direction of the main antenna 13 aligns with the longitudinal direction of the support 21, connecting the auxiliary antenna 30 to the main antenna 13, and then fitting a shell cover 18 around the inlet 11. The very small main antenna 13 has a slit 14 so as to form a stub for impedance matching. Moreover, the auxiliary antenna 30 enables communication distance to be increased in spite of the very small size of the inlet portion 10. For example, if a transmitted electric wave has a frequency of 2.45 GHz, a communication distance of several to several tens of centimeters can be expected.

A nonvolatile storage area of each IC chip 15 stores important data such as an ID which is uniquely assigned to the IC chip 15. However, since the biological implantation RFID tag 1 is implanted in the living body, it may need to be sterilized by subjecting it to ionizing radiation or a high temperature and a high pressure. Further, it is not preferable that the ID data be altered by a third person. Thus, the nonvolatile storage area of the IC chip 15 is preferably of a resistant, unrewritable ROM type in which, for example, important data is written during a semiconductor process so as to prevent data loss or alteration. Further, the shell cover 18, covering the biological implantation RFID tag 1, consists of a biocompatible material.

Fig. 2 is a plan view of the inlet 11, applied to the biological implantation RFID tag 1 (see Fig. 1) in accordance with the first embodiment of the present invention.

The inlet 11 is composed of a base film 12, the main antenna 13, and the IC chip 15.

The base film 12 is shaped like an elongate rectangle of width about 2.4 mm and consists of a dielectric such as resin (polyimide or the like). The main antenna 13, consisting of a conductor film such as copper or aluminum, is formed on the base film 12 by sticking a metal foil to the base film 12 or depositing metal on the base film 12. The main antenna 13 has a length of 6 mm and a width of 1.5 mm. The main antenna 13 has the slit 14 for impedance matching formed like an L-shaped key near the center thereof so as to form a stub contributing to impedance matching.

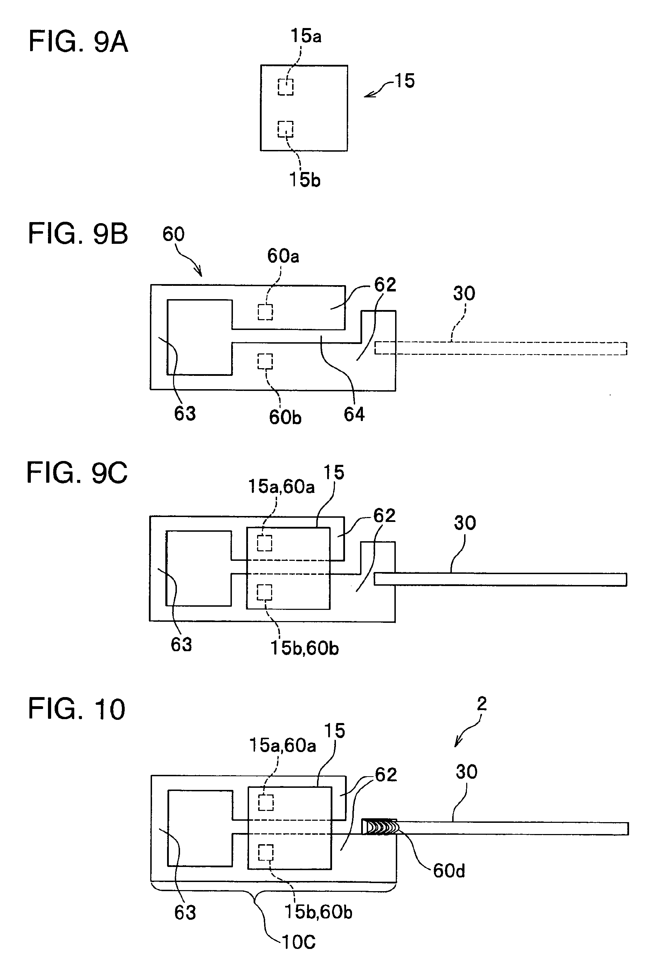

The IC chip 15 having 0.5 mm square with a thickness of about 0.1 mm is mounted in a bent portion of the slit 14. Two signal I/O electrodes (see Fig. 9A) of the IC chip 15 are composed of, for example, gold bumps and connected to the main antenna 13 across the slit 14. The signal I/O electrodes and main antenna 13 are connected together, for example, by ultrasonic junction or metal eutectic junction or with an anisotropic conductive film or a conductive adhesive.

As shown in Fig. 2, the main antenna 13 has the L-shaped slit 14, extending in the longitudinal direction (the length direction shown in the figure) through the substantial center of the main antenna 13 and bent at an end like a key so as to extend in a latitudinal direction (the width direction shown in the figure); the slit 14 is open along a longer side of the generally rectangle main antenna 13. The main antenna 13 with the slit 14 forms a generally rectangle stub enclosed by the slit 14. The stub and an area opposite the stub across the part of the slit 14 which extends in its latitudinal direction constitute a feeding portion for the main antenna 13. The IC chip 15 is connected to the feeding portion. Thus, the width of the slit 14 is slightly smaller than the distance between the two signal I/O electrodes of the IC chip 15.

Accordingly, the main antenna 13, the IC chip 15, and the stub formed by the slit 14 are connected in series. The stub operates as an inductance component of the series connection. The inductance component provided by the stub offsets a capacitance component of the IC chip 15, enabling the impedance matching between the feeding portion of the main antenna 13 and the two signal I/O electrodes of the IC chip 15.

The L-shaped slit 14 has been described. However, the main antenna 13 may have a T-shaped slit (not shown) extending in the longitudinal direction (the length direction shown in the figure) through the substantial center of the main antenna 13 and then in the latitudinal direction (the width direction shown in the figure) from the substantial center; the slit 14 is open along a longer side of the generally rectangle main antenna 13. In this example, two stubs are formed on the respective sides of the latitudinally extending part of the slit. Two areas located across the latitudinally extending part of the slit constitute a feeding portion to which the IC chip 15 is connected. The T-shaped slit exerts effects similar to those of the L-shaped slit 14.

Thus, the formation of the slit 14 in the main antenna 13 and thus the impedance matching stab enables the impedance matching between the main antenna 13 and the IC chip 15. This ensures a relatively long communication distance in spite of the very small size of the main antenna 13.

The base film 12 is formed of a resin material such as PET (polyethylene terephthalate) or PEN (polyester) which offers a heat sealing property. PET comprises PET in the base material to which a heat sealing resin sheet (not shown) is stuck or a special heat-sealing PET.

Fig. 3 is a perspective view of a support 21 to which the inlet 11 shown in Fig. 2 is stuck.

The support 21 is made of a dielectric such as resin (PET or PEN), ceramic, or rubber and is shaped like, for example, an elongate cylinder of diameter φ 0.8 mm. The inlet 11 shown in Fig. 1 is wrapped around the support 21 in the longitudinal direction. That is, the inlet 11 in Fig. 1 is wrapped around the elongate support 21 along its longitudinal direction as shown in Fig. 3. At this time, the base film 12 of the inlet 11 is wrapped around the outer periphery of the support 21 so that the widthwise opposite ends of the base film 12 do not overlap. The base film 12 can be temporarily bonded to the support 21 without heating. Then, the auxiliary antenna 30 is electrically connected and physically fixed, with a conductive adhesive, to a side of the inlet 11 attached to the support 21 which is opposite the slit 14. Thus, an inlet portion 10B shown in Fig. 4 is formed. The support 21 may have a diameter φ of 0.6 mm. In this case, when the inlet 11 is wrapped around the support 21, the widthwise opposite ends of the base film 12 overlap, whereas the widthwise opposite ends of the main antenna 13 do not contact each other.

Fig. 4 is a diagram showing the appearance of the biological implantation RFID tag 1B in accordance with the first embodiment of the present invention before it is covered.

As described above, the biological implantation RFID tag 1B is formed by sticking the inlet 11 shown in Fig. 2 to the support 21 shown in Fig. 3 to form the inlet portion 10B and attaching the auxiliary antenna 30 to the inlet 11. Applying a shell cover 18 to the biological implantation RFID tag 1B constitutes the biological implantation RFID tag 1 shown in Fig. 1.

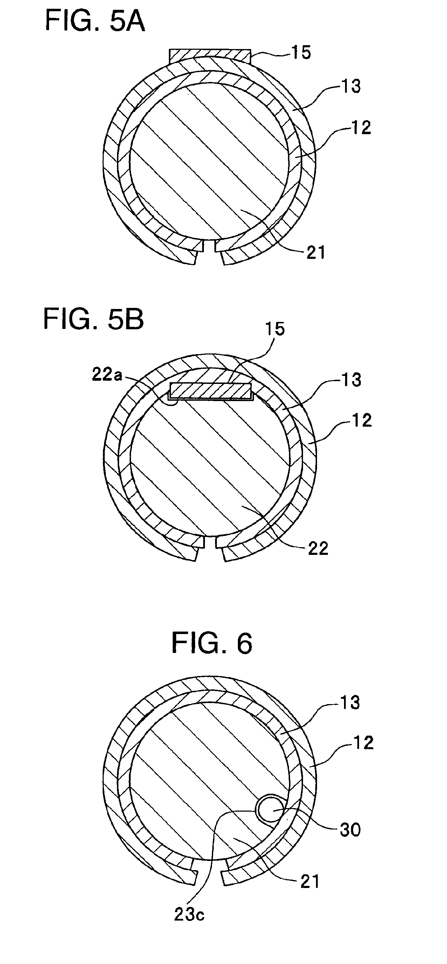

Fig. 5A is a sectional view taken along line A-A in Fig. 4 and showing the sectional shape of a first example of the biological implantation RFID tag 1B shown in Fig. 4. Fig. 5B is a sectional view taken along line A-A in Fig. 4 and showing the sectional shape of a second example of the biological implantation RFID tag 1B shown in Fig. 4.

In the first example, shown in Fig. 5A, as seen in a cross section of the biological implantation RFID tag 1B, that is, a cross section taken along line A-A in Fig. 4, the base film 12 is wrapped around the outer periphery of the support 21, with the main antenna 13 formed around the outer periphery of the base film 12 and the IC chip 15 mounted on the main antenna. Consequently, the cross section of the biological implantation RFID tag 1B is shaped to project by 0.1 mm, which corresponds to the thickness (height) of the IC chip 15.

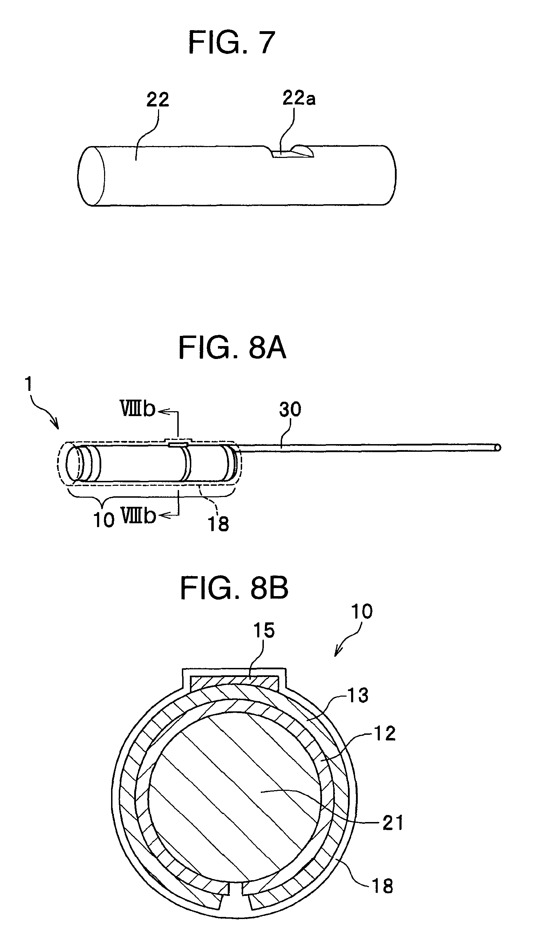

In the second example, the inlet 11 is wrapped around a support 22 having a recess 22a shown in Fig. 7 so that the IC chip 15 is located inside the inner periphery of the support 22 and so that the IC chip 15 is housed in the recess 22a, thereby preventing the IC chip 15 from projecting out.

Fig. 5B shows a cross section of the inlet portion 10B thus formed.

Fig. 6 is a sectional view taken along line B-B in Fig. 4 and showing the inlet portion 10B shown in Fig. 4. Fig. 6 shows an example of the connection between the main antenna 13 and the auxiliary antenna 30.

As shown in Fig. 6, a groove-like recess 23c having a generally circular cross section traversing the longitudinal direction is formed in the support 21. The auxiliary antenna 30 is fitted into the recess 23c. The auxiliary antenna 30 and the main antenna 13 are electrically connected and physically fixed to each other, for example, with a conductive adhesive. With the configuration shown in Fig. 5A (first example), the auxiliary antenna 30 may be connected to a surface of the main antenna 13, for example, with a conductive adhesive.

Specifically, the connection is made, for example, by the following procedure.
(1) The auxiliary antenna 30 (not covered with a biocompatible material described below) is connected to the main antenna 13 with a conductive adhesive and then covered with the biocompatible material.
(2) After the auxiliary antenna 30 is covered with the biocompatible material, a part of the cover which corresponds to a connection area is released. The auxiliary antenna 30 is then connected to the main antenna 13 with a conductive adhesive.
   The auxiliary antenna 30 and the main antenna 13 may be energized with the conductive adhesive or electrostatically coupled together so as to transmit signals of an operating frequency.
   Specifically, the connection is made by the following procedure.
(3) The auxiliary antenna 30 is fixed to a surface located opposite the main antenna 13 across the base film 12 so as to be electrostatically coupled to the main antenna 13. Moreover, if the auxiliary antenna 30 is not covered with a biocompatible material, it is covered with the biocompatible material after fixation.
   (3-1) If the inlet 11 (see Fig. 2) is wrapped around the support 21 so as to form a main antenna 13 around the outer periphery of the base film 12 as shown in Fig. 5A, the support 21 (having the groove-like recess 23c, into which the auxiliary antenna 30 is fitted) is used to pass the auxiliary antenna 30 through the inside of the base film 12.
   (3-2) If the inlet 11 (see Fig. 2) is wrapped around the support 21 so as to form the main antenna 13 inside the inner periphery of the base film 12 as shown in Fig. 5B, the support 22 shown in Fig. 5B (the groove-like recess 23c is unnecessary) is used to bond the auxiliary antenna 30 to the outside of the base film 12, that is, to the outermost periphery of the base film 12.
(4) The auxiliary antenna 30 is covered with a biocompatible material and then bonded to the main antenna 13 without releasing the cover. The auxiliary antenna 30 and the main antenna 13 are electrostatically coupled together.

The auxiliary antenna 30 consists of a thin, flexible conductor wire. The auxiliary antenna 30 can be manufactured using a unitary metal such as aluminum (A1), copper (Cu), silver (Ag), gold (Au), stainless steel, or titanium, or their alloy. Alternatively, a nonmetal conductor such as carbon fibers may be used. For reduced signal losses, the auxiliary antenna 30 is preferably thick. However, the present embodiment transmits signals of a high frequency in a 2.45 GHz band or the like to improve a skin effect. Consequently, from a practical viewpoint, the transmission capability does not depend significantly on the size of cross section of the auxiliary antenna 30. Thus, the auxiliary antenna 30 may be composed of a Litz wire.

Further, for reduced stress on small animals such as mice, the auxiliary antenna 30 is preferably as thin as possible. However, too small a thickness makes the auxiliary antenna 30 hard to handle or causes the auxiliary antenna 30 to be curled, entangled, or cut during operation. In view of these points, an appropriate value is set for the thickness.

The surface of the auxiliary antenna 30 may be covered with urethane or a material (biocompatible material) described below for the shell cover 18. This further reduces the stress applied to small animals by the auxiliary antenna 30 and makes it easier to handle.

Referring back to Fig. 1, the shell cover 18 is formed by passing the biological implantation RFID tag 1B formed as described above but still uncovered (see Fig. 4) through a heater cylinder (not shown), while wrapping a thermoplastic film consisting of PE (polyethylene), PP (polypropylene), elastomer, or the like around the outer periphery of the biological implantation RFID tag 1B. This allows the base film 12 of the inlet 11 to be firmly secured to the outer periphery of the support 21. The shell cover 18 is also thermally sealed on the biological implantation RFID tag 1B to entirely cover its outer periphery.

Alternatively, the shell cover 18 may be formed by placing and thermally sealing a tube-like shell material that is thermally contracted, around the outer periphery of the uncovered biological implantation RFID tag 1B.

Alternatively, the biological implantation RFID tag 1B may be covered with a covering material supplied in an aqueous form such as a silicone resin by immersing the inlet portion 10B in the aqueous covering material with a tip of the auxiliary antenna 30 gripped, lifting the inlet portion 10B from the material, and drying it.

Fig. 8 is a diagram showing the biological implantation RFID tag 1 manufactured in accordance with a variation of the first embodiment of the present invention. Fig. 8A is a perspective view of the biological implantation RFID tag 1. Fig. 8B is a sectional view of Fig. 8A taken along line C-C in Fig. 8A.

The biological implantation RFID tag 1 is formed by covering the uncovered biological implantation RFID tag 1B (see Fig. 4) with the shell cover 18 and then cutting the resulting tag 1B.

As shown in Fig. 8B, in the covered biological implantation RFID tag 1, the base film 12 is wrapped around the outer periphery of the support 21, and the main antenna 13 with the IC chip 15 mounted thereon is wrapped around the base film 12 and further covered with a shell cover 18. In this case, the projection of the IC chip 15 is accommodated by the shell cover 18, preventing the IC chip 15 from projecting outward markedly. As shown in Fig. 5B, fitting the IC chip 15 into the recess 22a in the support 22 prevents the IC chip 15 from projecting outward.

The shell cover 18 consists of a biocompatible resin or rubber material such as polyurethane, nylon, polyethylene, polystyrene, silicone rubber, thermoplastic fluorine resin, polyethylene terephthalate (PET), fluorine resin (Teflon (registered trade mark)), latex, hydrophilic polymer, polyurethane elastomer, or polyamide elastomer.

The above configuration enables a reduction in the size of the inlet portion 10 of the biological implantation RFID tag 1 shown in Fig. 1. For example, the inlet portion 10 has a diameter of about 1 mm and a length of about 7 mm.

Accordingly, if the biological implantation RFID tag 1 is subcutaneously implanted in a small animal such as a mouse via a subcutaneous implantation jig (for example, an injection needle), the size of the resulting invasive wound can be reduced, allowing the wound to be quickly cured, while inhibiting infection. Moreover, the very small size of a part of the biological implantation RFID tag 1B which enters the living body and the biocompatible material constituting the shell cover 18 places almost no stress on the small animal even when the biological implantation RFID tag 1 is subcutaneously implanted in the small animal. For example, the skin of the mouse is about 0.5 to 1.0 mm in thickness, but the biological implantation RFID tag 1 can be subcutaneously implanted easily in such a small animal with a thin skin.

The biological implantation RFID tag 1 in accordance with the present embodiment is very small and has a length of about 7 mm and a diameter of about 1 mm. The biological implantation RFID tag 1 is also covered with the biocompatible material. Consequently, the biological implantation RFID tag 1 can be implanted in the living body of a small animal such as a mouse with almost no stress placed on it. This prevents a possible reduction in the lifetime of the small animal.

Further, the inlet portion 10 with the IC chip 15 mounted thereon is implanted in the living body with the auxiliary antenna 30 located outside the living body. Thus, the auxiliary antenna 30 places almost no stress on the small animal. Furthermore, many signals are transmitted and received to and from the exterior of the living body through the auxiliary antenna 30 rather than through the living body. This improves communication performance.

### «Second Embodiment»

Fig. 9 is a diagram showing the assembly of a biological implantation RFID tag 2 in accordance with a second embodiment of the present invention.

In the biological implantation RFID tag 2, a metal lead frame 60 provides the functions of both the main antenna 13 (see Fig. 2) and support 21 (see Fig. 3) of the biological implantation RFID tag 1 in accordance with the first embodiment.

As shown in Fig. 9A, the IC chip 15 is of a single-side electrode type and has two signal I/O electrodes 15a and 15b located on the same surface thereof.

As shown in Fig. 9B, the lead frame 60 has a slit 64 formed along the longitudinal direction, a main antenna 62 formed from one end to the center thereof, and a U-shaped stub 63 for impedance matching formed at the other end thereof. The main antenna 62 includes a caulking portion 60d (see Fig. 10) for connection with the auxiliary antenna 30 and connecting portions 60a and 60b for connection with the two signal I/O electrodes 15a and 15b of the IC chip 15.

The size of the lead frame 60 is such that its length, width, and thickness are, for example, 1.5 mm, 0.8 mm, and 0.15 mm, respectively. The lead frame 60 consists of metal, for example, copper (Cu) or iron (Fe). The lead frame 60 can be formed by punching or etching a thin metal plate using a material commonly used for lead frames in the manufacture of semiconductor devices. Instead of the lead frame 60, a structure corresponding to the lead frame 60 may be formed in a thin metal film such as copper (Cu) or aluminum (Al) included in a film of a laminate structure using a resin film (not shown) such as a polyimide film as a reinforcement material. An RFID tag using the laminate film (not shown) is more flexible and thus less biologically invasive than the biological implantation RFID tag 2 using the lead frame 60. This is because the laminate film has a thickness of about 60 µm and is thus thinner than the lead frame 60, having a thickness of about 150 µm.

Then, as shown in Fig. 9C, the IC chip 15 is mounted on the main antenna 62. The two signal I/O electrodes 15a and 15b of the IC chip 15 and the main antenna 62 are connected together, for example, by ultrasonic junction or metal eutectic junction or with an anisotropic conductive film or a conductive adhesive.

Further, the auxiliary antenna 30 is connected to the main antenna 62. The connection between the auxiliary antenna 30 and the main antenna 62 is made in the same manner as that in which the IC chip 15 and the main antenna 62 are connected together or by caulking the auxiliary antenna 30 at an end of the main antenna 62.

Fig. 10 is a diagram showing the structure of the biological implantation RFID tag 2 in accordance with the second embodiment of the present invention formed as describe above. In this example, the auxiliary antenna 30 is caulked and connected to the caulking portion 60d of the main antenna 62.

The biological implantation RFID tag 2 is covered using the same material and treatment method as those in the first embodiment to form a shell cover 18 (see Fig. 1).

In the biological implantation RFID tag 2 in accordance with the second embodiment of the present invention, an inlet portion 10C to be implanted in the living body is very small and has a width of about 0.9 mm, a length of 1.7 mm, and a thickness of about 4 mm. The shape of the biological implantation RFID tag 2 in accordance with the second embodiment is such that its thickness and length are half and one-fifth, respectively, of those of the biological implantation RFID tag 1 in accordance with the first embodiment. This further facilitates the subcutaneous implantation of the biological implantation RFID tag in a small animal such as a mouse which has a thin skin.

### «Third Embodiment»

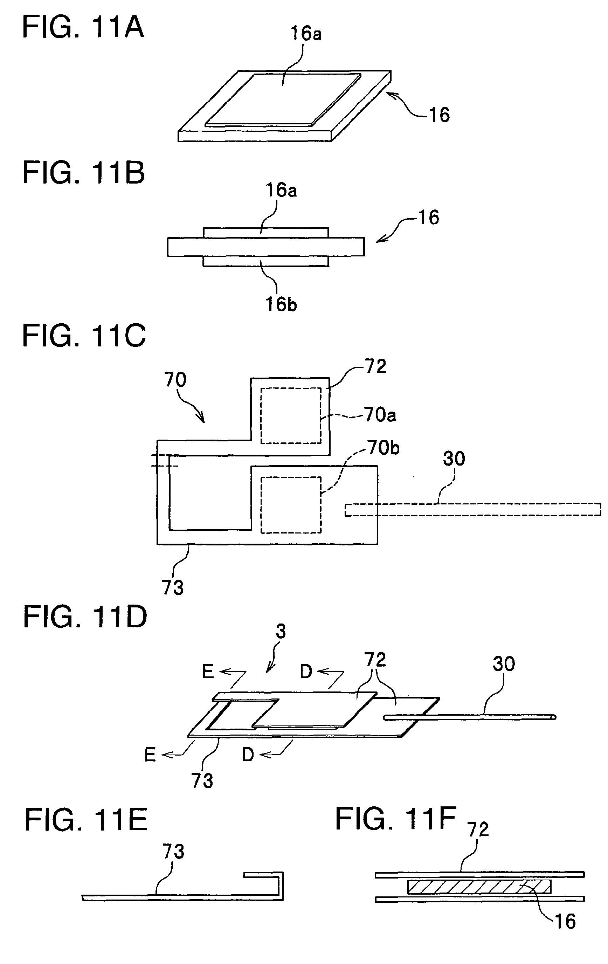

Fig. 11 is a diagram showing the assembly and configuration of a biological implantation RFID tag 3 in accordance with a third embodiment of the present invention.

The biological implantation RFID tag 3 in accordance with the third embodiment comprises an IC chip 16 of a double electrode type instead of comprising the IC chip 15 of a single electrode type in the biological implantation RFID tag 2 in accordance with the second embodiment. Further, the biological implantation RFID tag 3 in accordance with the third embodiment uses a lead frame 70 to provide the functions of both the main antenna 13 (see Fig. 2) and support 21 (see Fig. 3) of the biological implantation RFID tag 1 in accordance with the first embodiment, in almost the same manner as that in the second embodiment.

Fig. 11A is a perspective view of the IC chip 16. Fig. 11B is a side view of the IC chip 16.

The IC chip 16 is of a double electrode type and has a signal I/O electrode 16a formed on one surface (top surface) and a signal I/O electrode 16b formed on the other surface (bottom surface). The IC chip 16 may have the same electrical characteristics as those of the IC chip 15 (see Fig. 9A and the like) except for the arrangement of the electrodes. Either side of the IC chip 16 may be used as a front surface or a back surface for mounting, enabling a reduction in the number of assembly steps.

Fig. 11C is a plan view showing the shape of the lead frame 70.

The lead frame 70 has a main antenna 72, connected portions 70a and 70b for connection with the signal I/O electrodes 16a and 16b, and a stub 34b for impedance matching. The lead frame 70 is folded into two to sandwich the IC chip 16 between the folded portions to form an inlet portion 10F described below. When folded, the lead frame 70 has almost the same size as that of the lead frame 60 in accordance with the second embodiment and is 1.5 mm in length, 0.8 mm in width, and 0.15 mm in thickness. The material and forming method for the lead frame 70 may be the same as those for the lead frame 60 in accordance with the second embodiment.

Fig. 11D is a perspective view showing the appearance of the biological implantation RFID tag 3.

The lead frame 70 is folded into two to sandwich the IC chip 16 between the folded portions. The main antenna 72 is then mounted on the IC chip 16 to form an inlet. The two signal I/O electrodes 16a and 16b of the IC chip 16 and the connecting portions 70a and 70b of the main antenna 72 are connected together, for example, by ultrasonic junction or metal eutectic junction or with an anisotropic conductive film or a conductive adhesive.

Fig. 11E shows a cross section (E-E cross section) of the stub 34b for impedance matching. Fig. 11F shows a D-D cross section of the IC chip 16 after mounting.

Further, the auxiliary antenna 30 is connected to the main antenna 72 of the inlet to form a biological implantation RFID tag 3. The connecting method for and the configuration of the auxiliary antenna 30 may be the same as those in the second embodiment.

The shell cover 18 is wrapped around the biological implantation RFID tag 3 formed as described above. The covering material and treating method may be similar to those in the second embodiment.

The biological implantation RFID tag 3 thus formed has a very small inlet to be implanted in the living body; the inlet has a width of about 0.9 mm, a length of about 1.7 mm, and a thickness of about 0.4 mm. The size of the biological implantation RFID tag 3 is such that its thickness and length are half and one-fifth, respectively, of those of the biological implantation RFID tag 1 in accordance with the first embodiment.

The biological implantation RFID tag 3 in accordance with the third embodiment exerts the same effects as those of the biological implantation RFID tag 2 in accordance with the second embodiment. Further, owing to the use of the double electrode IC chip 16, the biological implantation RFID tag 3 in accordance with the third embodiment is effective for sharply reducing the required accuracy with which the IC chip 16 and main antenna 72 are mounted, improving the yield of the step of mounting the IC chip 16.

### « Fourth Embodiment»

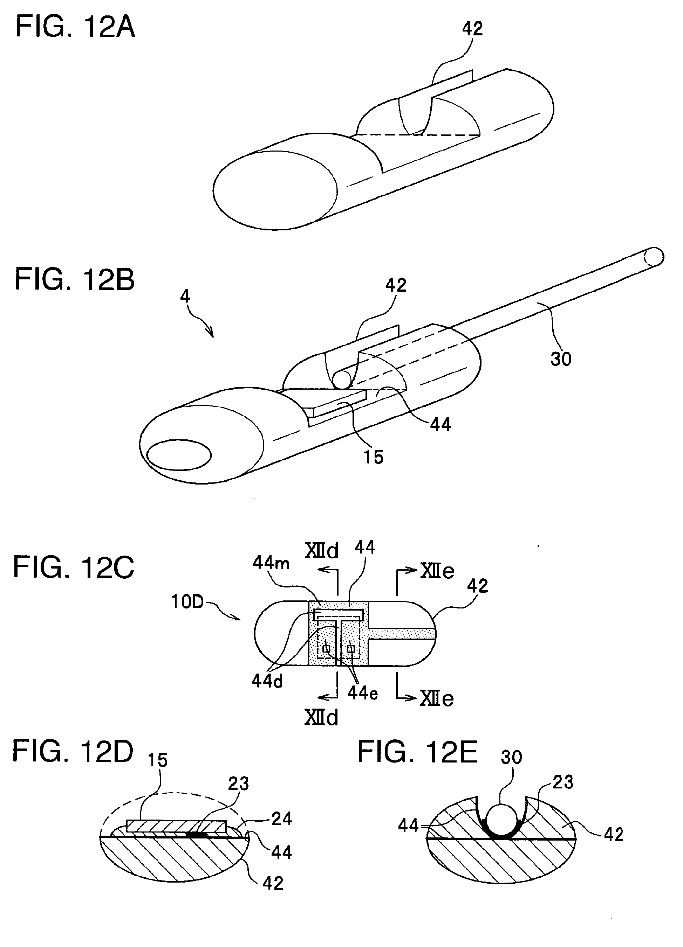

Fig. 12 is a diagram showing the assembly and configuration of a biological implantation RFID tag 4 in accordance with a fourth embodiment of the present invention.

As shown in Fig. 12A, a base frame 42 has an external shape like a cylinder, an elliptic cylinder, or a spheroid and has a planar portion formed by cutting away the center thereof in the longitudinal direction. The base frame 42 also has a groove-like portion formed so as to extend along the longitudinal direction from the planar portion to the exterior. The base frame 42 consists of a dielectric such as any of various resins or ceramic; the material is molded or machined into the above shape.

As shown in Fig. 12B, the biological implantation RFID tag 4 has an electrode 44 consisting of a conductor film and formed in the planar portion and groove-like portion of the base frame 42; the IC chip 15 is mounted on the electrode 44 and the auxiliary antenna 30 is connected to the IC chip 15.

The electrode 44 is formed by plating or depositing metal such as gold (Au) or aluminum (A1) on the base frame 42.

As shown in the plan view of the inlet portion 10D in Fig. 12C, the electrode 44 has a slit 44d, a main antenna element 44e, and an impedance matching circuit 44m.

As shown in an F-F cross section of the inlet portion 10D in Fig. 12D, the IC chip 15 is of a single electrode type and is physically fixed to the base frame 42 with an adhesive material 24. Two signal I/O electrodes are electrically connected to a connecting portion of the electrode 44 with a conductive material 23.

As shown in a G-G cross section of the inlet portion 10D in Fig. 12E, one end of the auxiliary antenna 30 is fitted into the groove-like portion of the base frame 42 and mechanically fixed and electrically connected to the electrode 44 with the conductive material 23.

A shell cover (not shown) is further wrapped around the biological implantation RFID tag 4 described above.

The biological implantation RFID tag 4 in accordance with the fourth embodiment not only exerts the effects of the above embodiments but also facilitates manufacture through the use of the base frame 42.

### «Fifth Embodiment»

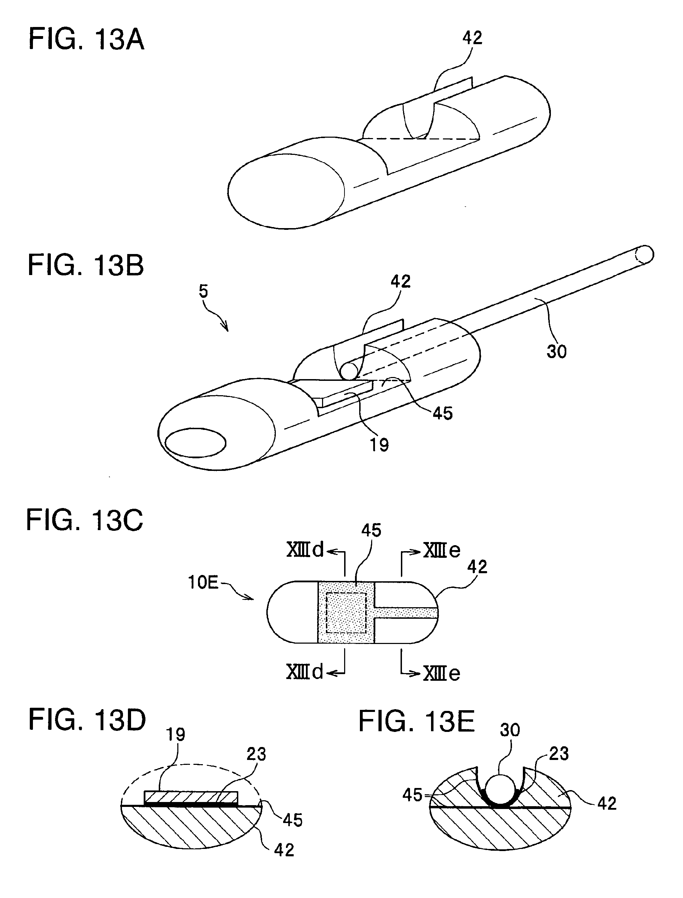

Fig. 13 is a diagram showing the assembly and configuration of a biological implantation RFID tag 5 in accordance with a fifth embodiment of the present invention.

As shown in Fig. 13A, the base frame 42 has an external shape like a cylinder, an elliptic cylinder, or a spheroid and has a planar portion formed by cutting away the center thereof in the longitudinal direction. The base frame 42 also has a groove-like portion formed so as to extend along the longitudinal direction from the planar portion to the exterior. The base frame 42 consists of a dielectric such as any of various resins or ceramic; the material is molded or machined into the above shape.

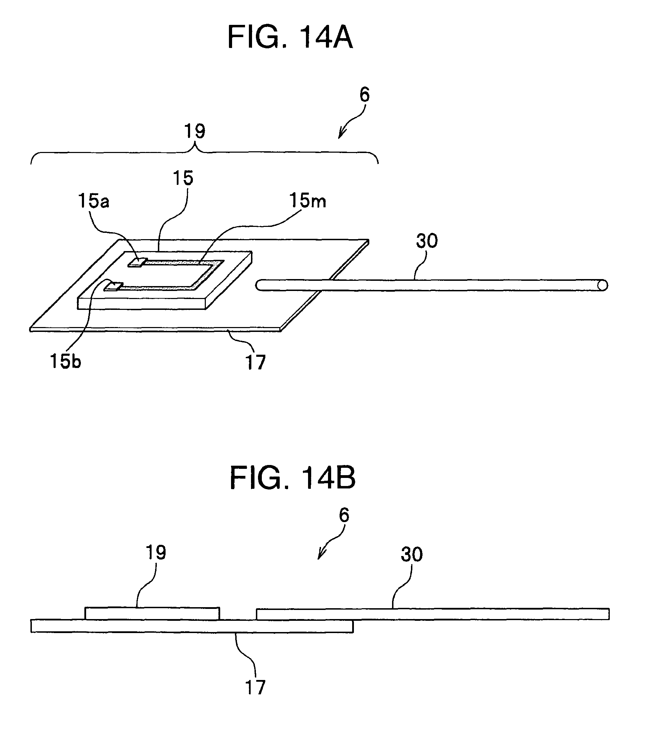

As shown in Fig. 13B, the biological implantation RFID tag 5 has an electrode 45 composed of a conductor film and formed in the planar portion and groove-like portion of the base frame 42, an IC chip 19 of a single electrode type (described below with reference to Fig. 14) mounted on the electrode 45 and having the IC chip 15 and a matching circuit 15m provided thereon, and the auxiliary antenna 30 connected to the electrode 45.

The electrode 45 is formed by plating or depositing metal such as gold (Au) or aluminum (Al) on the base frame 42.

As shown in the plan view of the inlet portion 10E in Fig. 13C, the electrode 45 does not need to have any special pattern.

As shown in an F-F cross section of the inlet portion 10E in Fig. 13D, the IC chip 19 has one surface physically fixed and electrically connected to the electrode 44 with the conductive material 23.

Further, as shown in a G-G cross section of the inlet portion 10E in Fig. 13E, one end of the auxiliary antenna 30 is fitted into the groove-like portion of the base frame 42 and mechanically fixed and electrically connected to the electrode 44 by the conductive material 23.

A shell cover (not shown) is further wrapped around the biological implantation RFID tag 5 described above.

The biological implantation RFID tag 5 in accordance with the fifth embodiment not only exerts the effects of the biological implantation RFID tag 5 in accordance with the fourth embodiment but also enables a reduction in steps by using the IC chip 19 of a single electrode type having the IC chip 15 and the matching circuit 15m provided thereon.

### «Sixth Embodiment»

Fig. 14 is a diagram showing the structure of a biological implantation RFID tag 6 in accordance with a sixth embodiment of the present invention. Fig. 14A is a perspective view of the biological implantation RFID tag 6. Fig. 14B is a side view of the biological implantation RFID tag 6.

The biological implantation RFID tag 6 has a plate-like main antenna 17 composed of a conductor similar to the lead frame 60 (see Fig. 10B), the IC chip 19 having the IC chip 15 of a single electrode type (see Fig. 10A) and the matching circuit 15m provided thereon, the IC chip 19 being mounted on the main antenna 17 so that the signal I/O electrodes 15a and 15b are located on the top surface (lying opposite a surface that is in contact with the main antenna 17), and the auxiliary antenna 30 connected to an end of the main antenna 17.

The IC chip 19 and auxiliary antenna 30 are physically fixed and electrically connected to the main antenna 17 using the above material (not shown) and method.

The signal I/O electrodes 15a and 15b of the IC chip 15 provide unbalanced outputs; one of the signal I/O electrodes 15a and 15b is grounded inside the IC chip 15, whereas the other is electrically connected to the main antenna 17.

The top surface of the IC chip 19 except for the positions of the signal I/O electrodes 15a and 15b has, for example, an oxide film or an insulating layer formed thereon so as to be electrically insulated from the IC chip body 15.

The top surface of the IC chip 19 has the matching circuit 15m formed thereon and composed of a conductor line such as metal to electrically connect the signal I/O electrodes 15a and 15b together. The matching circuit 15m is typically U-shaped but may have another shape such as the letter M or a spiral so as to - exhibit desired electrical characteristics. The conductor line of the matching circuit 15m is set to have to an appropriate electrical length so as not to extend out from the top surface of the IC chip 19.

A cover may also be placed on the biological implantation RFID tag 6 as described above.

The biological implantation RFID tag 6 in accordance with the sixth embodiment of the present invention comprises the IC chip 19 having the IC chip 15 and the matching circuit 15m provided thereon. This enables a further reduction in the size of the biological implantation RFID tag.

### «Seventh Embodiment»

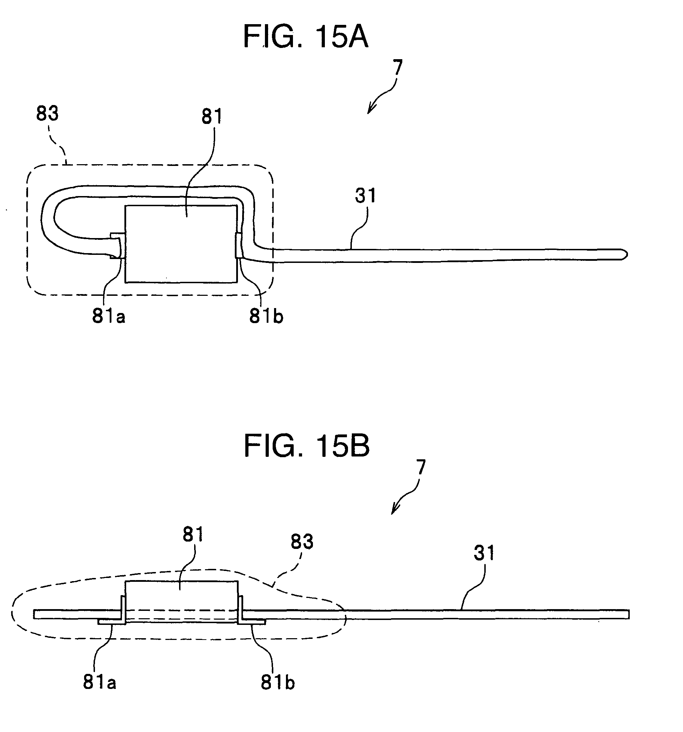

Fig. 15 is a perspective view showing a biological implantation RFID tag 7 in accordance with a seventh embodiment of the present invention. Fig. 15A is a plan view of the biological implantation RFID tag 7. Fig. 15B is a side view of the biological implantation RFID tag 7.

The biological implantation RFID tag 7 comprises an IC package 81, an antenna 31, and a covering material 83.

The IC package 81 has a chip similar to the IC chip 15 (see Fig. 9A) or IC chip 16 (see Fig. 11A) described above and an I/O circuit provided thereon and sealed with a package. The IC package 81 has signal I/O electrodes 81a and 81b.

The antenna 31 may be configured similarly to the auxiliary antenna 30 (see Fig. 1 and the like).

The covering material 83 may be made of a material similar to that of the shell cover 18 (see Fig. 1 and the like).

For the biological implantation RFID tag 7, the antenna 31 is produced by, first, connecting its start end to the signal I/O electrode 81a of the IC package 81, drawing it by a predetermined length, connecting its middle to the signal I/O electrode 81b, and cutting it so as to leave a predetermined length.

Consequently, the section from the signal I/O electrode 81a to signal I/O electrode 81b of the antenna 31 operates similarly to the stub 63 (see Fig. 9), the stub 73 (see Fig. 10), or the matching circuit 15m (see Fig. 13).

Further, the section from the signal I/O electrode 81b to terminal of the antenna 31 operates similarly to the auxiliary antenna 30 (see Fig. 1).

The IC package 81 and the section from the signal I/O electrode 81a to signal I/O electrode 81b of the antenna 31 are sealed with a covering material 83. The covering material 83 is made of a material similar to that of the shell cover 18 (see Fig. 1 and the like).

In the example shown in the figures, the section from the signal I/O electrode 81a to signal I/O electrode 81b of the antenna 31 passes through side surfaces of the IC package 81. However, this section may pass on the bottom surface of the IC package 81.

The biological implantation RFID tag 7 in accordance with the seventh embodiment of the present invention allows products to be completed with a sharply reduced number of steps.

### «Eighth Embodiment»

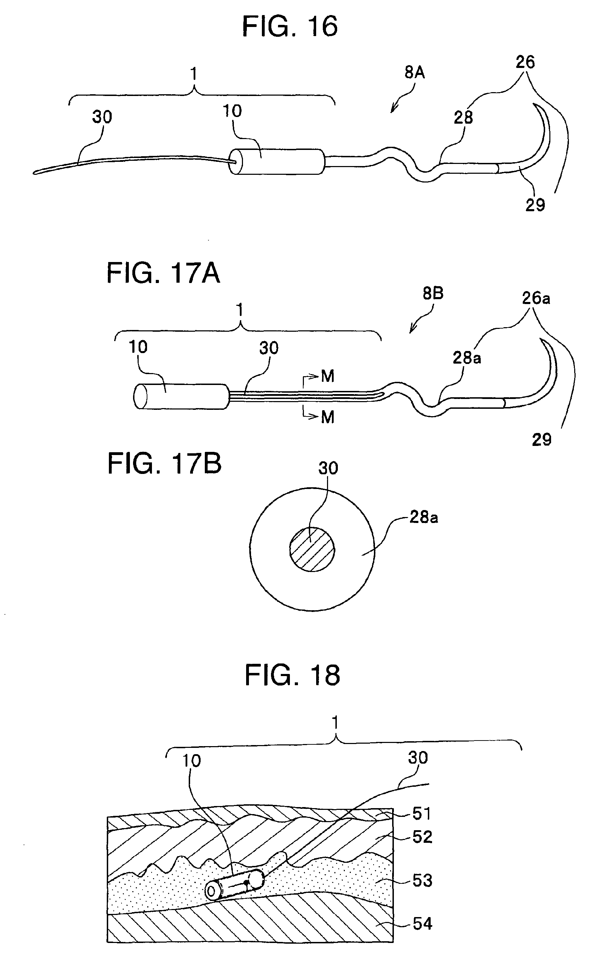

Fig. 16 is a diagram showing the configuration of an insertion jig 8A for a biological implantation RFID tag in a first example of an eighth embodiment of the present invention (see Figs. 1 and 8 as required).

As shown in Fig. 16, the insertion jig 8A for a biological implantation RFID tag includes a suture with a needle 26 coupled to the biological implantation RFID tag 1, having the inlet portion 10 to be subcutaneously implanted in a small animal.

Any of the biological implantation RFID tags 2 to 7 in accordance with the second to seventh embodiments may be used in place of the biological implantation RFID tag 1 in accordance with the first embodiment.

Specifically, first, the biological implantation RFID tag 1B without the shell cover 18, shown in Fig. 8A, is produced. Then, a material for the tube-like shell cover 18 is cut to a length slightly larger than that of the biological implantation RFID tag 1. The biological implantation RFID tag 1 is then inserted into the resulting material of the shell cover 18. Further, an end of the suture 28 is inserted from one end of the material of the shell cover 18 by a predetermined length. This allows the suture 28 to be inserted from one end the biological implantation RFID tag 1 installed in the shell cover 18. The material for the shell cover 18 contracts thermally, and the shell cover 18 with the suture 28 installed therein is caulked and temporarily joined to the biological implantation RFID tag 1. The outer periphery of the shell cover 18 is uniformly heated to thermally contract and seal the material of the shell cover 18.

As shown in Fig. 16, the biological implantation RFID tag 1 and the suture with the needle 26 can be integrally combined together to form the biological implantation RFID tag 1 shown in Fig. 1. A heat sealing process using, for the suture 28, a thermoplastic material similar to that of the shell cover 18 enables a more effective covering process, allowing an increase the mounting strength of the suture with the needle 26.

Any commercially available medical or dental suture may be used as the suture with the needle 26 provided that a suture needle 29 is pre-attached to one end of the suture 28.

The needle 29 has a length and a curvature both suitable for subcutaneous implantation in small animals. For example, the needle 29 with the suture 28 has a sharp end with an inverted triangular cross section, is generally steeply bent, and has a length of 18 mm and a thickness of 0.43 mm. The inverted triangular cross section means that a plane (the base of the triangular in the cross section) is formed along the inner periphery of the bent portion of the needle 29 and that a cut surface (the vertex of the triangle in the cross section) is formed along the outer periphery of the bent portion.

The needle 29 can be subcutaneously inserted to an appropriate depth to allow the biological implantation RFID tag 1B to be appropriately implanted so as to lie along the skin.

After the biological implantation RFID tag 1 is subcutaneously inserted into a small animal using the suture with the needle 26, the biological implantation RFID tag 1 can be prevented from moving in the body by knotting in the suture. The knot of the suture enables the visual check of the position where the biological implantation RFID tag 1 is implanted.

Fig. 17 is a diagram showing the configuration of an insertion jig 8B for a biological implantation RFID tag in a second example of an eighth embodiment of the present invention (see Figs. 1 and 8 as required).

The insertion jig 8B in the second example shown in Fig. 17 includes a suture 28a having a core extending along its longitudinal direction and the auxiliary antenna 30 installed in the core as shown in Fig. 17A. Thus, first, the auxiliary antenna 30 of the biological implantation RFID tag 1 is penetratingly inserted through the suture 28a. Then, the biological implantation RFID tag 1B and suture 28a coupled together are inserted into the material of the tube-like shell cover 18 (see Fig. 1). Further, the outer periphery of the shell cover 18 is uniformly heated to thermally contract and seal the material of the shell cover 18. Fig. 17B is an M-M cross section of the auxiliary antenna 30 installed in the suture 28a.

As shown in Fig. 17A, the biological implantation RFID tag 1 can thus be integrally coupled to a suture with a needle 26a composed of the suture 28a containing the auxiliary antenna 30 and the needle 29, to form the biological implantation RFID tag 1 shown in Fig. 1. A heat sealing process using, for the suture 28a, a thermoplastic material similar to that of the shell cover 18 enables a more effective covering process, allowing an increase in the mounting strength of the suture with the needle 26a.

Fig. 18 is a sectional view showing the structure of the skin of a common animal.

In the skin of an animal, an epidermal layer 51, a dermal layer 52, and a subcutaneous tissue layer 53 are laminated in this order from the surface to interior of the body. A muscle 54 exists under the subcutaneous tissue layer 53. The thickness of the skin generally refers to the sum of the thickness of - the epidermal layer 51 and the thickness of the dermal layer 52. The thickness of the skin of a small animal such as a mouse (that is, the sum of the thicknesses of the epidermal layer 51 and dermal layer 52) is 0.5 to 1.0 mm.

Then, insertion jigs 8A and 8B for a biological implantation RFID tag in accordance with an eighth embodiment of the present invention enables any of the small biological implantation RFID tags 1 to 7 with a diameter of 1 mm and a length of 7 mm or less to be accurately implanted in the subcutaneous tissue layer 53 of a small animal such as a mouse.

### «Ninth Embodiment»

A biological implantation RFID tag in accordance with a ninth embodiment of the present invention (not shown) applies a cover of a tissue adhesive material to the auxiliary antenna 30 and antenna 31 of any of the biological implantation RFID tags 1 to 7 in accordance with the first to seventh embodiments. In the description below, the tissue adhesive material is applied to the biological implantation RFID tag 1 in accordance with the first embodiment. However, the tissue adhesive is also applicable to any of the biological implantation RFID tags 2 to 7 in accordance with the second to seventh embodiments.

When the biological implantation RFID tag 1 (see Fig. 1) is subcutaneously implanted in a small animal, the inlet portion 10 is implanted in the subcutaneous tissue layer 53 with the auxiliary antenna 30 passing through the dermal layer 52 and epidermal layer 51 and partly projecting out from the body surface. Movement of the living body or the part of the auxiliary antenna 30 located outside the living body may increase the size of an exit in the living body from which the auxiliary antenna 30 projects. Then, bacteria may invade the body through the exit. Thus, the tissue adhesive material is used to cover the inlet portion 10 of the biological implantation RFID tag 1 and the area of the auxiliary antenna 30 which is in contact with the living body. This prevents the possible release of the tissue resulting from movement of the living body or the part of the auxiliary antenna 30 located outside the living body. The traceability of the biological implantation RFID tag 1 can thus be improved.

To prevent the invasion of bacteria from the exterior, a member consisting of a biocompatible material such as ceramics is placed in a part of the living body. This improves the adhesion of the biological implantation RFID tag to the living tissue. However, in spite of their biocompatibility, ceramics cannot follow the flexible movement of the living body owing to their hardness. Consequently, movement of the living body may release the biological implantation RFID tag from the tissue.

The tissue adhesive material is preferably cellulose or a cellulose derivative, more preferably the cellulose or cellulose derivative to which heparin or cell adhesive protein is attached, the cellulose or cellulose derivative which is alkali activated, or the cellulose derivative with charge. Further, the tissue adhesive material is preferably heparin or cell adhesive protein. The member consisting of the tissue adhesive material is preferably selected from the group consisting of a non-woven cloth, a film, and a porous member or is preferably formed by covering the auxiliary antenna 30 with the tissue adhesive material.

The auxiliary antenna 30 configured as described above is pre-covered with, for example, silicone rubber, thermoplastic fluorine resin, fluorine rubber, polytetrafluoroethylene (PTFE), polyethylene, polyplopyrene, polyvinyl chloride, polyethylene terephthalate (PET), polystyrene, or polyurethane, particularly, with a thermoplastic material. In terms of biocompatibility, in particular, silicone rubber is preferably used. In terms of machinability, thermoplastic fluorine resin is preferably used. To improve biocompatibility, for example, polyurethane elastomer, polyamide elastomer, unplasticized vinyl chloride resin, silicone rubber, or the like may be attached to the surface of the auxiliary antenna 30.

Alternatively, cellulose and a cellulose derivative such as cellulose acetate, carboxymethyl cellulose, cellulose-N, N-diethyl aminoethyl ether (DEAE cellulose) can be preferably used as a tissue adhesive material.

When the cellulose or cellulose derivative is used as a tissue adhesive material, it is preferable to attach heparin, tissue adhesive protein, or the like to the surface of the cellulose or cellulose derivative, to subject the surface to a treatment such as alkali activation, or to use a cellulose derivative with charge. This configuration provides several preferable results; it facilitates granulation and cell derivation and improves biocompatibility and adhesion strength.

The method for attaching heparin or tissue adhesive protein to the cellulose or cellulose derivative or subjecting the cellulose or cellulose derivative to alkali activation is not limited, and any of various well-known methods may be used. The member formed of the tissue adhesive material is preferably located under the dermis of the living wall.

A preferable tissue adhesive material is cellulose or a cellulose derivative such as cellulose acetate or carboxymethyl cellulose.

The biological implantation RFID tag in the ninth embodiment of the present invention improves the adhesion of the inlet portion 10 and auxiliary antenna 30 to the living tissue in the area of the adhesion. Thus, even when the body, the auxiliary antenna 30, or the like moves, the biological implantation RFID tag can follow the flexible movement without being released from the tissue. Thus, the biological implantation RFID tag can preferably prevent bacteria or the like from invading the body.

### <<Tenth Embodiment>>

A package for a biological implantation RFID tag in accordance with a tenth embodiment of the present invention is in the form of a package that accommodates any of the biological implantation RFID tags 1 to 7 in accordance with the first to seventh embodiment for distribution.

As described above, the biological implantation RFID tags 1 to 7 have a diameter of about 1 mm and a length of about 7 mm and is thus very small; handling the biological implantation RFID tag requires attention.

Thus, the package for the biological implantation RFID tag is in the form in which any of the biological implantation RFID tags 1 to 7 installed in a syringe is contained in a blister pack, so as to be subcutaneously implanted easily in a small animal such as a mouse.

With the package for the biological implantation RFID tag in accordance with the tenth embodiment of the present embodiment, the user may take the syringe out of the blister and insert it into a small animal. This allows the user to easily handle the biological implantation RFID and prevents the user from carelessly touching the biological implantation RFID tags 1 to 7. As a result, the biological implantation RFID tag can be hygienically inserted.

### <<Comparative Example>>

If a long frequency band of about 130 to 200 kHz is used as a communication frequency, its wavelength is about 1,500 m, which is much longer than the wavelength used in the embodiments of the present invention. To transmit or receive signals of such a long frequency band via a biological implantation RFID tag of length about 12 mm and diameter about 2 mm, it is necessary to form an antenna coil by winding a copper wire around a ferrite or iron core several hundred times. Consequently, the biological implantation RFID tag in this comparative example includes the antenna coil composed of the ferrite or iron core and the copper wire and which is thus very heavy and inflexible. As a result, when implanted in a small animal such as a mouse, the biological implantation RFID tag in the comparative example, which is large and massive, significantly stresses the small animal to reduce its lifetime.

Owing to its very small size, the biological implantation RFID tag in accordance with the present invention can be effectively used in research institutes for biology or pathology to control experiments on small animals such as mice.

It should be further understood by those skilled in the art that although the foregoing description has been made on embodiments of the invention, the invention is not limited thereto and various changes and modifications may be made without departing from the spirit of the invention and the scope of the appended claims.

## Claims

1. An RFID tag to be inserted into a living body of an animal so that an electromagnetic wave is transmitted between the RFID tag and an outside of the living body to read an identification data for identifying the RFID tag from the RFID tag, comprising:
an inlet portion including a dielectric substrate, an IC chip (15) storing therein the identification data and mounted on the substrate, and a main antenna (13, 17, 62, 72) connected to the IC chip, and
an auxiliary antenna (30, 31) connected electrically to the main antenna (13, 17, 62, 72).

2. An RFID tag to be inserted into a living body of an animal so that an electromagnetic wave is transmitted between the RFID tag and an outside of the living body to read an identification data for identifying the RFID tag from the RFID tag, comprising:
in inlet portion including an IC chip (15) storing therein the identification data, and an electrically conductive lead frame (60, 70) having a main antenna (13, 17, 62, 72) connected to the IC chip (15), and
an auxiliary antenna (30, 31) connected electrically to the main antenna (13, 17, 62, 72).

3. The RFID tag according to claim 2, wherein the IC chip has signal input and output electrodes (16a, 16b) formed on respective surfaces of the IC chip opposite to each other so that the lead frame (70) is bent to extend on both of the surfaces in the inlet portion.

4. The RFID tag of any preceding claim, further comprising an exterior cover (18, 83) of biocompatibility covering the inlet portion.

5. The RFID tag of any of claims 1 to 3, further comprising an exterior cover of biocompatibility, wherein the inlet portion has a cylindrical shape covered by the exterior cover.

6. The RFID tag of claim 4 or 5, wherein the inlet portion and the exterior cover are joined to each other by thermal welding.

7. The RFID tag of any preceding claim, wherein the auxiliary antenna (30, 31) extends from the main antenna so that at least a part of the auxiliary antenna (30, 31) projects from the living body when the inlet portion is arranged in the living body.

8. The RFID tag of any preceding claim, wherein the main antenna includes an impedance matching circuit (63, 73, 15m) for impedance matching between the IC chip and a combination of the main and auxiliary antennas.

9. The RFID tag according to claim 8, wherein the main antenna has a slit (14, 64, 44d) of L-shape or T-shape to form a stub forming the impedance matching circuit (63, 73, 15m).

10. The RFID tag of claim 2 or any claim dependent on claim 2, wherein the lead frame has a stub of U-shape to form an impedance matching circuit.

11. The RFID tag of any preceding claim, further comprising an electrically conductive wire of U-shape arranged on the IC chip electrically isolated, both ends of which electrically conductive wire are connected to the IC chip to form an impedance matching circuit.

12. The RFID tag of claim 1 or any claim dependent on claim 1, wherein the substrate is a film of flat shape or curved shape.

13. The RFID tag of claim 1 or any claim dependent on claim 1, wherein the substrate is of cylindrical shape or spheroidal shape.

14. The RFID tag of claim 1 or any claim dependent on claim 1, wherein the main antenna includes an electrically conductive layer formed on the substrate.

15. The RFID tag of claim 14, wherein the electrically conductive layer includes a slit to form a stub forming an impedance matching circuit.

16. The RFID tag of any preceding claim, wherein a longitudinal length of the inlet portion is not more than 10% of a body length of the animal, and a transverse length of the inlet portion is not more than 1.5% of the body length of the animal.

17. The RFID tag of any preceding claim, wherein a body length of the animal is not more than 10 cm, a longitudinal length of the inlet portion is not more than 7 mm, and a transverse length of the inlet portion is not more than 1 mm.

18. The RFID tag of claim 1 or any claim dependent on claim 1, wherein the substrate has a recess for receiving a projected part of the IC chip.

19. The RFID tag of claim 1 or any claim dependent on claim 1, wherein the substrate has a groove extending in a longitudinal direction of the substrate to receive a projected part of the IC chip.

20. The RFID tag of any preceding claim, wherein the IC chip has a non-volatile memory of ROM type not rewritable.

21. The RFID tag of any preceding claim, further comprising an adhesive capable of adhering to the living body of the animal.

22. A jig comprising:
the RFID tag according to any preceding claim, and
a surgical suture jointed with the RFID tag and having a needle for inserting the RFID tag into the living body of the animal so that the electromagnetic wave is transmitted between the RFID tag and the outside of the living body to read the identification data for identifying the RFID tag from the RFID tag.

23. The jig of claim 22, wherein the auxiliary antenna extends in the surgical suture.
